Europäisches Patentamt

European Patent Office

Office européen des brevets

(19)

(11) **EP 1 291 460 A1**

(12) **EUROPEAN PATENT APPLICATION**
published in accordance with Art. 158(3) EPC

(43) Date of publication:
**12.03.2003 Bulletin 2003/11**

(21) Application number: **01932316.1**

(22) Date of filing: **29.05.2001**

(51) Int Cl.$^7$: **D06M 13/342**, D06M 15/15,
A61L 15/28, A61L 15/42,
A61F 13/15, A61F 13/20,
A61F 13/36, A61F 13/47,
C08B 3/14, A61F 5/441,
A61L 2/16
// D06M101:06

(86) International application number:
**PCT/JP01/04493**

(87) International publication number:
**WO 01/092632 (06.12.2001 Gazette 2001/49)**

(84) Designated Contracting States:
**AT BE CH CY DE DK ES FI FR GB GR IE IT LI LU
MC NL PT SE TR**

(30) Priority: **30.05.2000 JP 2000159774
22.08.2000 JP 2000251070**

(71) Applicant: **Ajinomoto Co., Inc.
Tokyo 104-8315 (JP)**

(72) Inventors:
• **KURAUCHI, Masahiko,
c/o Amino Science Laboratories
Kawasaki-shi, Kanagawa 210-8681 (JP)**

• **FURUTA, Kiyonori,
c/o Amino Science Laboratories
Kawasaki-shi, Kanagawa 210-8681 (JP)**
• **SATO, Hiroyuki, c/o Amino Science Laboratories
Kawasaki-shi, Kanagawa 210-8681 (JP)**

(74) Representative: **Nicholls, Kathryn Margaret et al
MEWBURN ELLIS
York House
23 Kingsway
London WC2B 6HP (GB)**

(54) **FIBER PRODUCT HAVING ANTIBACTERIAL AND DEODORANT FUNCTION**

(57)    According to the present invention, a cellulose fiber or a fiber product made of said cellulose fiber is contacted with a basic amino acid ester and thereafter heat treated, thereby there may be provided an antibacterial product which does not lose its antibacterial property even by being washed several times.

The fiber product is excellent in antibacterial property, deodorant property and safety and suitable for use as an antibacterial product such as socks, towel or the like which especially an odor becomes a problem due to the propagation of bacteria during using or storing it, and further it is suitable for use as a medical or sanitary article.

**EP 1 291 460 A1**

**Description**

Technical Field

The present invention relates to a fiber product which is excellent in antibacterial property, deodorant property, durability to washing and safety. More particularly, it relates to a fiber product comprising a partial ester of a basic amino acid with cellulose and/or a salt thereof or to a fiber product comprising a processed cellulose fiber which may be obtained by contacting a cellulose fiber with a basic amino acid and thereafter heat-treating the mixture.

Also, the present invention relates to the use as a medical or sanitary article of the fiber product which is excellent in antibacterial property and safety.

Background Art

Since the propagation of bacteria during using or storing a fiber product causes often unpleasant results such as odor, etc., an antibacterial processing has been hitherto applied to cloth products such as socks, towel and the like where especially odor becomes problem. In addition, numerous and new antibacterial products have been put on the marketplace in a variety of fields under the background that the infection of human being by methicillin-resistant Staphylococcus aureus (MRSA) and infectious bacillus subtilis O-157 became recently a social problem. Further, with respect to medical or sanitary articles such as a paper diaper, an incontinence pad, a physiological napkin, a physiological tampon, a panty sheet, a sweat pad, a breast milk pad, a medical tampon, an applicator, a first-aid adhesive plaster, a wet tissue, a surgical dressing, a medical mask, a gauze, a bandage, a medical sheet, a medical towel, a medical drape, a gown for a patient who receives an operation, a cloth for a patient, a medical cap, a medical apron, a medical cover and the like, in considering that these articles are directly contacted with a skin, a mucous membrane or a wound and absorb the liquids secreted, exuded or excreted from the living body during use, thereby becoming an environment where microorganisms are liable to propagate and that they are practically used in medical places such as operation, etc. where bacterial contamination is disliked exceedingly, the antibacterial property is considered to be one of important function from the standpoint of prevention of an infection.

With respect to a means to impart an antibacterial property into these fiber products, if socks are taken as an example to be targeted, Japanese Patent Application Open-Laid Nos. Sho 61-231202 and Sho 63-249701 disclose socks which comprise used cupper as an antibacterial agent. Also, Japanese Patent Application Open-Laid No. Hei 3-249201 discloses antibacterial and deodorant socks which comprises used a polyvalent metal in combination with a quaternary ammonium salt. Japanese Patent Application Open-Laid No. Hei 5-49682 discloses socks which comprise used a mixture of aluminum oxide, silicon oxide and magnesium oxide as an antibacterial agent. Japanese Patent Application Open-Laid No. Hei 6-287811 discloses raw yarns for socks which comprise used dissoluble glass containing silver as an antibacterial agent.

Among them, however, the methods using cupper, a polyvalent metal or silver have the problems that there is a possibility of causing an allergy during use due to these metals released and eluted by water, sweat or the like and further that these metals are also released from socks by washing, etc., thereby the effect cannot be maintained. Also, since the method using aluminum oxide, silicon oxide and magnesium oxide as antibacterial agents is one wherein the antibacterial property is imparted into fibers by kneading said antibacterial agents with fibers, it has a problem that these antibacterial agents are released by washing, etc., thereby the effect cannot be maintained.

As an attempt to impart the antibacterial property into medical or sanitary articles, a paper diaper containing benzalkonium chloride as an antibacterial agent is disclosed in Japanese Patent Application Open-Laid No. Hei 4-2345. A sanitary article containing silver-carried strongly cationic exchange resin composed of sulfonic acid radical-introduced polystyrene resin as an antibacterial agent is disclosed in Japanese Patent Application Open-Laid No. Hei 5-212094. Body liquid absorbent articles containing chlorhexidine hydrochloride or domiphen bromide as an antibacterial agent is disclosed in Japanese Patent Application Open-Laid No. Hei 9-10296. Sanitary physiological articles containing cyclodextrin-encapsulated phytoncide as antibacterial agent is disclosed in Japanese Patent Application Open-Laid No. Hei 9-108261. Sanitary articles containing silver-carried carboxymethylcelulose as antibacterial agent is disclosed in Japanese Patent Application Open-Laid No. Hei 11-1895. An antibacterial sheet containing a water soluble plant extract containing an aminated rosin and sanitary articles made of said antibacterial sheet are disclosed in Japanese Patent Application Open-Laid No. Hei 11-200245.

However, as the problem which is common to these methods disclosed, a part of the product involved therein contains certain medicine or metal each having the antibacterial property in any case and the mode contained of these antibacterial ingredients is due to admixture, adhesion or ionic bond so that they are eluted by the liquids secreted, exuded or excreted from the living body or otherwise they release by friction with the skin, mucous membrane or wound, thereby there is a possibility of causing an allergy, etc.

Disclosure of the Invention

An object of the present invention is to provide a fiber product which has at least one of effects that it is excellent in antibacterial property, deodorant property, durability to washing and safety.

Another object of the present invention is to provide a fiber product which is excellent in antibacterial property and safety and which is suitable as a medical or sanitary article.

The present inventors have found that the above objects can be solved by using a specific cellulose ester, a cellulose fiber subjected to the specific treatment or a fiber product made of said cellulose fiber, and have completed the present invention.

That is, the present invention is a fiber product which is characterized by containing a partial ester of a basic amino acid with cellulose and/or a salt thereof.

Also, the present invention is a fiber product which is characterized by containing a processed cellulose fiber which may be obtained by contacting a cellulose fiber with a basic amino acid ester and thereafter heat treating the mixture, and further it is a fiber product obtained by spinning, weaving or twisting the blend of said processed cellulose fiber with other fiber.

Also, the present invention intends the use as a medical or sanitary article or a part of these articles a fiber product made of a partial ester of a basic amino acid with cellulose and/or a salt thereof which itself has antibacterial property. That is, it relates to the use of a fiber product made of a partial ester of a basic amino acid with cellulose and/or a salt thereof as a medical or sanitary article.

As the partial ester of a basic amino acid with cellulose takes a structure of forming covalent bond (ester bond) between a carboxyl group of a basic amino acid and a hydroxyl group of cellulose, its possibility to be desorbed by water, sweat or the like is low and hence its durability to washing is high. Even when it was used as a medical or sanitary article, its possibility to be eluted with the liquids secreted, exuded or excreted from the living body or to be released by friction with the skin, mucous membrane or wound is low. Also, even when the ester bond was cut, the component released is an amino acid which is harmless to the living body, and therefore the safety is very high.

The partial ester of a basic amino acid with cellulose and/or a salt thereof for use in the present invention has a structure wherein at least one of hydroxyl groups present in the cellulose molecule is esterified with a basic amino acid. This basic amino acid may be mixture of several kinds of ones. Also, there may be used one wherein an esterification degree is 0.00001-3.

The basic amino acid moiety in the partial ester of a basic amino acid with cellulose and/or a salt thereof for use in the present invention is the residue of lysine, arginine, ornithine or histidine which becomes correspondingly a partial ester of lysine with cellulose, a partial ester of arginine with cellulose, a partial ester of ornithine with cellulose or a partial ester of histidine with cellulose. In this case, the basic amino acid may be mixed amino acids.

The fiber product for use in the present invention which is characterizing by containing the partial ester of a basic amino acid with cellulose or a salt thereof can be prepared by the following method. That is, a cellulose fiber or a fiber product made of the cellulose fiber is first allowed to contact with a treating solution containing a basic amino acid ester. When required, an excess solution may be removed from the impregnated fiber or fiber product and the wet fiber or fiber product may be dried. And then, the dried fiber is subjected to a heat treatment. Subsequently, after-treatment such as washing or the like for removing the unreacted basic amino acid is conducted. The cellulose for use here may also be cotton pre-treated with alkali or the like according to a usual method. Furthermore, by using an optional acid in the steps after heat treatment, the basic amino acid residue bonded with cellulose fiber may be converted into its salt with the optional acid. As the kind of a salt, there may be taken a salt with an organic acid such as acetate, lactate, malate, succinate, citrate, benzoate, pyrrolidone carboxylate or the like; a salt with a mineral acid such as hydrochloride, sulfate, phosphate or the like; or a salt with Lewis acid such as zinc chloride.

The cellulose fiber obtained by the above method may be spun, weaved or twisted in blend with other fiber, for example a chemical fiber such as nylon, polyester, acryl or the like; and a natural fiber such as wool, silk or the like according to the conventional method, thereby the fiber product of the present invention may also be obtained.

Incidentally, the cellulose fiber obtained by the above method may be any form of cotton wool, raw yarn, raw cloth or articles after sewing.

As the treating solution, there is used one wherein a basic amino acid ester, preferably lower alkyl ester having 1-6 carbon atoms represented by methyl ester has been dissolved in water, alcohol or a mixture thereof. In the case that the basic amino acid ester is in the form of a salt with hydrochloric acid, sulfuric acid or the like, it may be neutralized with 10 to 200 mol % of sodium hydroxide or the like based on the basic amino acid depending on the necessity. The content of the basic amino acid in the treating solution may be optional, if it is such a range that it is dissoluble or dispersible therein. The cellulose fiber is immersed in the treating solution, the excess solution is removed from the impregnated fiber depending on the necessity, and thereafter an air-drying or a drying under heating is conducted when required. The dried fiber is heat treated at 100-200 °C, preferably 120-180 °C for 1-100 minutes, preferably 2-60 minutes and the objected product may be prepared through washing and drying steps. Although washing is conducted in order of firstly water, subsequently an aqueous alkaline solution such as sodium bicarbonate or the like, an aqueous acidic solution such as citric acid or the like and finally water, a part of them may be omitted when required. The fiber product in the present invention is a general term including a thread, a felt, an unwoven cloth, a mat and the like, and specifically includes clothing such as an undershirt, a shirt, a blouse, stockings, a coat, a sweater, a cardigan, a jacket, trousers, a skirt, tights, a gymnastics wear, sports clothes, a T-shirt, a trainer, wristlet, pajamas, a negligee, a bathrobe, socks, gloves, a tie, a scarf, a muffler, a hat, a white robe and an apron; bedclothes such as a cover for thick bed quilt, a blanket, a pillowcase and a sheet; in addition a towel, a bath towel, a bath mat, a kitchen mat, a cloth for toilet stool, a toilet mat, a dishcloth, handkerchiefs, a cloth diaper, a carpet, a curtain, etc. In the case that a fiber form or a processing form during the antibacterial processing by a basic amino acid derivative is in the stage before the final product after sewing as taken here, an appropriate additional processing for finishing the final product may be conducted.

In the present invention, the fiber product made of the partial ester of a basic amino acid with cellulose and/ or a salt thereof can be preferably used as a medical or sanitary article. Specifically, a cloth or an unwoven cloth as the form of the partial ester of a basic amino acid with cellulose and/or a salt thereof may be disposed as a part of the article to the area to be contacted, particularly the skin, mucous membrane or wound, and the hitherto known structure may also be adopted to other part of the article. In case of a medical or sanitary article designed so as to absorb liquids secreted, exuded or excreted from the living body such as a paper diaper, an incontinence pad, a physiological napkin, a physiological tampon, a panty sheet, a sweat pad, a breast milk pad, a medical tampon, an applicator, a first-aid adhesive plaster, a surgical dressing and the like, the partial ester of a basic amino acid with cellulose and/or a salt thereof may be disposed to the absorbent or a part thereof in addition to the overall constituent material of the article. In this case, the form is not necessarily a cloth or an unwoven cloth, and it may be fibrous or powdery.

The Best Mode For Carrying Out The Invention

The present invention is illustrated in more detail by the preparation examples and the examples but it does not restricted to these examples.

Preparation Example 1 < preparation of L-lysine cellulose partial ester citrate >

2.33 Grams (10 mmol) of L-lysine methyl ester dihydrochloride was dissolved in 15 ml of methanol, and 5 ml of 2 N aqueous sodium hydroxide solution was added thereto to make a treating solution. 5.0 Grams of a cotton unwoven cloth (C×32, a product of Hanilon Co., Ltd.) was immersed in this treating solution for about 30 seconds and air-dried for a hour, and thereafter heat treated at 140 °C for 20 minutes. It was washed with water, and further a washing by 5 % aqueous sodium bicarbonate solution-3 times of rinsing by water and a washing by 10 % aqueous citric acid solution-3 times of rinsing by water were conducted, and thereafter dehydration and air-drying were conducted to make a sample. After a part of the sample was dried at 50 °C over a night under a vacuum, about 0.5 g weighed accurately and stirred at room temperature for 18 hours in 50 ml of 0.5 N aqueous sodium hydroxide solution to effect alkaline hydrolysis. The fiber was filtered out and the determination of L-lysine was conducted with an amino acid analyzer (L-8500, Hitachi Co., Ltd.).

From the result, the amount of the bonded L-lysine based on 1 g of the sample was calculated to be 0.105 mmol (an esterification degree of 0.017). Also, from the result of the determination for citric acid present in the same test solution was conducted with a HPLC, the amount of the bonded citric acid based on 1 g of the sample was calculated to be 0.190 mmol.

Preparation Example 2 < preparation of L-arginine cellulose partial ester citrate>

A similar experiment as in Preparation Example 1 was conducted except that 2.61 g (10 mmol) of L-arginine methyl ester dihydrochloride was used in place of L-lysine methyl ester dihydrochloride. As a result of the calculation for the amount of the bonded arginine based on 1 g of the sample in the similar method as in Preparation Example 1, it was 0.109 mmol (an esterification degree of 0.018). Also, similarly the amount of the bonded citric acid based on 1 g of the sample was 0.162 mmol.

Example 1: preparation of socks

5.83 Grams (25 mmol) of L-lysine methyl ester dihydrochloride was dissolved in 20 ml of methanol, and 12.5 ml of 2 N aqueous sodium hydroxide solution was added thereto to make a treating solution. Socks (about 25 g) made of pure cotton was immersed in this treating solution for about 20 seconds and air-dried for 2 hours, and thereafter heat-treated at 140 °C for 20 minutes. The heat-treated socks were first washed with water, further washed with 5 % aqueous sodium bicarbonate solution, rinsed 3 times with water, washed with 10 % aqueous citric acid solution, rinsed 3 times with water, further washed with household neutral washing detergent solution and rinsed with water. And thereafter it was dehydrated and air-dried to obtain the socks of the present invention.

Experimental Example 1: determination of the bonded amount of an amino acid

About 0.5 g of a cut piece was prepared from the socks obtained in Example 1 and dried at 50 °C over a night in a vacuum desiccator wherein phosphorous pentaoxide was placed as a desiccating agent. This sample weighed accurately and then was stirred at room temperature for 18 hours in 50 ml of 0.5 N aqueos sodium hydroxide solution to effect an alkaline hydrolysis. After fiber was filtered out, the determination of L-lysine was conducted with an amino acid analyzer (L-8500, Hitachi Co., Ltd.).
From the result, the amount of the bonded L-lysine based on 1 g of the sample was calculated to be 0.106 mmol.

Experimental Example 2: antibacterial test

A cut piece of square having about 18 mm in a side was prepared from the socks obtained in Example 1 and an antibacterial test was conducted according to a standardized test method provided by Japanese Association for the Function Evaluation of Textile. Staphyrococus aures (ATCC 5638P) was employed as the test bacteria. The specimen which has been subjected to high pressure vapor sterilization was seeded with bacteria (about $2.6 \times 10^4$) suspended in Nutient Broth culture medium and the cultivation was conducted at 37 °C for 18 hours, and thereafter the viable count was measured. Also, this socks was washed 5 times according to the process described in "JIS L 0127 Item 103" and thereafter the seed and cultivation were similarly conducted to measure the viable count. Incidentally, a nylon white cloth was used as the standard white cloth.
The bacteriostatic activity and bactericidal activity were calculated according to the following equations described in JIS L 1902: 1998.

$$S = M_b - M_c$$

$$L = M_a - M_c$$

wherein S, L, $M_a$, $M_b$ and $M_c$ represent the bacteriostatic activity, the bactericidal activity, a constant logarithm for viable count on the untreated sample immediately after seed (an average of three specimens), a constant logarithm value for viable count on the untreated sample after cultivation for 18 hours (an average of three specimens), a constant logarithm for viable count on the treated sample after cultivation for 18 hours (an average of three specimens), respectively.
The results of the antibacterial test for the socks are shown in table 1.

Table 1

| Sample | Viable Count | Bacteriostatic Activity | Bactericidal Activity |
|---|---|---|---|
| Socks of Example (Before washing) | <20 | >6.0 | > 3.1 |
| Socks of Example (After 5 times of washing) | <20 | >6.0 | >3.1 |
| Nylon white cloth (immediately after seeding) | $2.6 \times 10^4$ | - | - |
| Nylon white cloth (after cultivation for 18 hours) | $1.8 \times 10^7$ | - | - |

As shown in table 1, the numerical values for the socks of Example 1 meet the standard values for the antibacterial and deodorant product provided by Japanese Association for the Function Evaluation of Textile. It was confirmed from this result that the socks prepared in Example 1 show sufficient antibacterial property and simultaneously the duability to washing.

Experimental Example 3: wearing test

Each of 3 men's panels (A-C) put on untreated socks to his one side of foot and put on the socks prepared in Example 1 to his other side of foot for 8 hours, respectively and thereafter there was conducted with respect to odor, sweat absorbance and wear feeling the sensory evaluation in 3 stages (◎ : no odor, a good sweat absorbance and a good wear feeling, ○: slight odor, an ordinary sweat absorbance and an ordinary wear feeling, ✕: a strong odor, a bad sweat absorbance and a bad wear feeling). The results are shown in table 2.

Table 2

| Evaluation Item | Panel A | | Panel B | | Panel C | |
|---|---|---|---|---|---|---|
| | Example | Untreated | Example | Untreated | Example | Untreated |
| Odor | ○ | ✕ | ○ | ✕ | ○ | ✕ |
| Sweat Absorbance | ○ | ○ | ○ | ○ | ○ | ○ |
| Wear Feeling | ○ | ○ | ○ | ○ | ○ | ○ |

It was confirmed from these tests that the socks prepared by Example 1 has an effect to reduce an odor without impairing their sweat absorbance and wear feeling.

Experimental Example 4: elution test

0.405 Grams of the cut piece of the socks prepared in Example 1 was shaken in 10 ml of pure water at room temperature to conduct an elution test. As a result of the analysis for the eluted component with a HPLC, it was recognized that 0.0094 mmol of L-lysine based on 1 g of the cut piece after the test for 1 hour was eluted and 0.0115 mmol of L-lysine based on 1 g of the cut piece after the test for 1.5 hours was eluted. Other eluted component than L-lysine was not recognized.

Example 2

Medical masks were respectively prepared using the unwoven cloth made of L-lysine cellulose partial ester citrate of Preparation Example 1 and the unwoven cloth made of L-arginine cellulose partial ester citrate of Preparation Example 2. Also, as a comparative product a medical mask was similarly prepared using an untreated unwoven cloth.

Experimental Example 5

The sensory evaluation by 5 expert panels of the feeling during wearing these medical masks was made in 5 stages wherein (1) the feeling is very bad, (2) the feeling is bad, (3) the feeling is ordinary, (4) the feeling is good and (5) the use feeling is very good. There was no difference in the evaluation point among the medical masks prepared respectively using the unwoven cloth made of L-lysine cellulose partial ester citrate and the unwoven cloth made of L-arginine cellulose partial ester citrate and the medical mask prepared using the untreated unwoven cloth.

Experimental Example 6

A cut piece of square having about 18 mm in a side was each prepared from these medical masks and was subjected to the antibacterial test according to the process described in "JIS L 1902: 1998 (Antibacterial activity test method for fibrous products 8, a quantitative test)". Staphyrococus aures ATCC 5638P and Klebsiella pneumoniae ATCC 4532 were employed as the test bacteria. Each of the specimens was subjected to high pressure vapor sterilization and thereafter seeded with the prescribed amount (about $2.5 \times 10^4$) of bacteria suspended in Nutient Broth culture medium and the cultivation was conducted at 37 °C for 18 hours, and thereafter the respective viable counts were measured. The results are shown in tables 3 and 4. Also, the proliferation value, the bacteriostatic activity and bactericidal activity were calculated from the equations $F = M_b - M_a$, $S = M_b - M_c$ and $L = M_a - M_c$ which are described in JIS L 1902: 1998 wherein F: the proliferation value, S: the bacteriostatic activity, L: the bactericidal activity, $M_a$: a constant logarithm for viable count on the untreated sample immediately after seed (an average of three specimens), $M_b$: a constant logarithm value for viable count on the untreated sample after cultivation for 18 hours (an average of three specimens) and $M_c$: a constant logarithm for viable count on the treated sample after cultivation for 18 hours (an average of three specimens). The results are shown in tables 3 and 4.

Table 3

| Test Bacteria (Deposit Number) | Staphylococcus aureus (ATCC 6538P) | Klebsiella pneumoniae (ATCC 4352) |
|---|---|---|
| Concentration of Bacteria Seeded | $2.5 \times 10^4$ | $2.5 \times 10^4$ |
| Viable Count after 18 hours (number/ml) | <20 | <20 |
| Proliferation Value (F) | 2.8 | 3.3 |
| Bacteriostatic Activity (S) | >5.9 | >6.4 |
| Bactericidal Activity (S) | >3.1 | >3.1 |

Table 4

| Test Bacteria (Deposit Number) | Staphylococcus aureus (ATCC 6538P) | Klebsiella pneumoniae (ATCC 4352) |
|---|---|---|
| Concentration of Bacteria Seeded | $2.5 \times 10^4$ | $2.5 \times 10^4$ |
| Viable Count after 18 hours (number/ml) | $1.9 \times 10^2$ | <20 |
| Proliferation Value (F) | 2.8 | 3.3 |
| Bacteriostatic Activity (S) | 4.9 | >6.4 |
| Bactericidal Activity (S) | 2.1 | >3.1 |

It was confirmed from these tests that the medical masks prepared respectively using the unwoven cloth made of L-lysine cellulose partial ester citrate and the unwoven cloth made of L-arginine cellulose partial ester citrate have sufficient antibacterial property.

Experimental Example 7

0.422 Grams of the cut piece of the medical mask prepared using the unwoven cloth made of L-lysine cellulose partial ester citrate was shaken in 10 ml of water at room temperature to conduct an elution test. As a result of the analysis for the eluted component with a HPLC, it was recognized that 0.0098 mmol of L-lysine based on 1 g of the cut piece after the test for 1 hour was eluted and 0.0130 mmol of L-lysine based on 1 g of the cut piece after the test for 1.5 hours was eluted. Other eluted component than L-lysine was not recognized.

Industrial Applicability

According to the present invention, it became possible to provide a fiber product having at least one of the effects of being excellent in antibacterial property, deodorant property, durability to washing and safety, said fiber product being made of a cellulose fiber into which an antibacterial property has been imparted by contacting with a basic amino acid ester and the subsequent heat treatment. Said fiber product is preferably used as especially a medical or sanitary article owing to its excellent antibacterial property and safety.

**Claims**

1. A fiber product which is **characterized by** containing a partial ester of a basic amino acid with cellulose and/or a salt thereof.

2. A fiber product which is **characterized by** containing a processed cellulose fiber which may be obtained by contacting a cellulose fiber with a basic amino acid ester and thereafter heat treating the mixture.

3. A fiber product which is **characterized by** containing spun, weaved or twisted yarn or its cloth in the blended form with a processed cellulose fiber which may be obtained by contacting a cellulose fiber with a basic amino acid ester and thereafter heat treating the mixture.

4. The fiber product as claimed in any one of claims 1 to 3 wherein the basic amino acid is lysine, arginine, ornithine or histidine.

5. The fiber product as claimed in any one of claims 1 to 4 wherein the cellulose is cotton.

6. The use of the fiber product as claimed in any one of claims 1 to 5 as a medical or sanitary article.

7. The use of the fiber product as a medical or sanitary article as claimed in claim 6 wherein the medical or sanitary article is a paper diaper, an incontinence pad, a physiological napkin, a physiological tampon, a panty sheet, a sweat pad, a breast milk pad, a medical tampon, an applicator, a first-aid adhesive plaster, a wet tissue, a surgical dressing, a medical mask, a gauze, a bandage, a medical sheet, a medical towel, a medical drape, a gown for patient who receives an operation, a cloth for patient, a medical cap, a medical apron or a medical cover.

8. A medical or sanitary article containing the fabric product as claimed in any one of claims 1 to 5.

9. The medical or sanitary article as claimed in claim 8 wherein the medical or sanitary article is a paper diaper, an incontinence pad, a physiological napkin, a physiological tampon, a panty sheet, a sweat pad, a breast milk pad, a medical tampon, an applicator, a first-aid adhesive plaster, a wet tissue, a surgical dressing, a medical mask, a gauze, a bandage, a medical sheet, a medical towel, a medical drape, a gown for patient who receives an operation, a cloth for patient, a medical cap, a medical apron or a medical cover.

## INTERNATIONAL SEARCH REPORT

| International application No. |
| --- |
| PCT/JP01/04493 |

**A. CLASSIFICATION OF SUBJECT MATTER**
Int.Cl$^7$   D06M13/342, D06M15/15, A61L15/28, A61L15/42, A61F13/15, A61F13/20,
A61F13/36, A61F13/47, C08B3/14, A61F5/441, A61L2/16 // D06M101:06

According to International Patent Classification (IPC) or to both national classification and IPC

**B. FIELDS SEARCHED**

Minimum documentation searched (classification system followed by classification symbols)
Int.Cl$^7$   D06M13/342, D06M15/15, A61L15/28, A61L15/42, A61F13/15, A61F13/20,
A61F13/36, A61F13/47, C08B3/14, A61F5/441, A61L2/16

Documentation searched other than minimum documentation to the extent that such documents are included in the fields searched
Jitsuyo Shinan Koho          1926-1996     Toroku Jitsuyo Shinan Koho   1994-2001
Kokai Jitsuyo Shinan Koho   1971-2001     Jitsuyo Shinan Toroku Koho   1996-2001

Electronic data base consulted during the international search (name of data base and, where practicable, search terms used)
WPI/L

**C. DOCUMENTS CONSIDERED TO BE RELEVANT**

| Category* | Citation of document, with indication, where appropriate, of the relevant passages | Relevant to claim No. |
| --- | --- | --- |
| A | JP 3-199470 A (Ajinomoto Co., Inc.),<br>30 August, 1991 (30.08.91),<br>Full text   (Family: none) | 1-5 |
| A | US 4077771 A (Tomio KUROKI, Teramae KAZUHIRO),<br>07 May, 1978 (07.05.78),<br>Full text<br>& JP 52-12396 A | 1-5 |
| A | JP 11-113780 A (Asahi Chemical Industry Co., Ltd.),<br>27 April, 1999 (27.04.99),<br>Full text   (Family: none) | 1-9 |
| A | JP 9-132869 A (Chisso Corporation),<br>20 May, 1997 (20.05.97),<br>Full text   (Family: none) | 1-9 |

☐ Further documents are listed in the continuation of Box C.          ☐ See patent family annex.

| | |
| --- | --- |
| * Special categories of cited documents:<br>"A" document defining the general state of the art which is not considered to be of particular relevance<br>"E" earlier document but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |
| Date of the actual completion of the international search<br>23 July, 2001 (23.07.01) | Date of mailing of the international search report<br>07 August, 2001 (07.08.01) |
| Name and mailing address of the ISA/<br>Japanese Patent Office | Authorized officer |
| Facsimile No. | Telephone No. |

Form PCT/ISA/210 (second sheet) (July 1992)